(19) Europäisches Patentamt
European Patent Office
Office européen des brevets

(11) Publication number: **0 406 760 B1**

(12) **EUROPEAN PATENT SPECIFICATION**

(45) Date of publication of patent specification: **05.10.94**

(51) Int. Cl.5: **A01N 37/52**, C07C 257/00

(21) Application number: **90112585.6**

(22) Date of filing: **02.07.90**

The file contains technical information submitted after the application was filed and not included in this specification

(54) **Plant growth regulating composition.**

(30) Priority: **03.07.89 JP 172633/89**
**03.07.89 JP 172634/89**

(43) Date of publication of application:
**09.01.91 Bulletin 91/02**

(45) Publication of the grant of the patent:
**05.10.94 Bulletin 94/40**

(84) Designated Contracting States:
**CH DE FR GB IT LI NL**

(56) References cited:
**EP-A- 411 199**

**PATENT ABSTRACTS OF JAPAN, unexamined applications, C field, vol. 14, no. 149, March 22, 1990 THE PATENT OFFICE JAPANESE GOVERNMENT page 55 C 705**

**CHEMICAL ABSTRACTS, vol. 97, no. 23, December 6, 1982 Columbus, Ohio, USA UBE INDUSTRIES LTD. "N-Phenyl-amidinecarboxylic acids" page 557, column 1, abstract-no. 197 999q**

(73) Proprietor: **SUMITOMO CHEMICAL COMPANY, LIMITED**
**Kitahama 4-chome 5-33**
**Chuo-ku Osaka 541 (JP)**

(72) Inventor: **Izumi, Kazuo**
**15-10-104, Kusunoki-cho**
**Asiya, Hyogo (JP)**
Inventor: **Katoh, Tsuguhiro**
**2-10-2-244, Sonehigashi-cho**
**Toyonaka, Osaka (JP)**
Inventor: **Muranaka, Toshiya**
**2-10-4-411, Sonehigashi-cho**
**Toyonaka, Osaka (JP)**
Inventor: **Sanemitsu, Yuzuru**
**2-2-924, Wakaba-cho**
**Ashiya, Hyogo (JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**CHEMICAL ABSTRACTS**, vol. 90, no. 9, February 26, 1979 Columbus, Ohio. USA Y. SUGIYAMA et al. "N-Aryl- amidinoformic acids" page 490, column 1, abstract-no. 71 933a

**CHEMICAL ABSTRACTS**, vol. 89, no. 25, December 18, 1978 Columbus, Ohio, USA Y. SUGIYAMA et al. "Acid-catalyzed reaction of ethyl cyanoformate with aromatic amines in acetic acid: facile synthesis of N-substituted amidinoformic acids and ethyl 4-quinazolone-2-carboxylate" page 560, column 1, abstract-no. 215 037r

**CHEMCIAL ABSTRACTS**, vol. 78, no. 11, March 19, 1973 Columbus, Ohio, USA J. YOSHIMURA et al." N-substituted carbamimidoylformic acids" page 418, column 2, abstract-no. 71 503n

## Description

The present invention relates to a plant growth regulating composition. More particularly, it relates to a plant growth regulating composition comprising as the active ingredient an amidinoformic acid derivative or its salt.

It is known that secondary metabolites (e.g. coumarins, flavonoids, chlorogenic acid, lignins, phenolic compounds) produced in plants via the shikimic acid pathway play important roles therein, for instance, as pigmentary substances or lignified substances. However, the presence of these metabolites are sometimes weary or troublesome for the purpose of utilization of plants on a human life. For instance, chlorogenic acid in sunflower seeds is conjugated with proteins therein to make the seeds blackish or deteriorate the seed taste, thereby lessen their quality as food. Further, for instance, lignin is contained in trees in several ten percents but takes no part in paper production, and its elimination from pulp is one of the essential routine operations.

One of the known methods for controlling or suppressing the production of said secondary metabolites in plants is to inhibit the activity of phenylalanine ammonia-lyase (hereinafter referred to as "PAL"). PAL is an enzyme which initiates the deamination of phenylalanine to give trans-cinnamic acid and is known to control the rate of the biosynthesis of secondary metabolites via the shikimic acid pathway. Among known PAL-inhibitors, 2-aminoxy-3-phenylpropionic acid (hereinafter referred to as "AOPP") (Phytochemistry, 27, 711 (1988)) is considered as the most effective, but its inhibitory effect is still not satisfactory.

JP-A-57/85352, as described in Chemical Abstracts 97: 197999 and Derwent Abstract No. 82-55839E, discloses that certain N-phenylamidinecarboxylic acids are useful as fungicides and insecticides.

Through a diversified investigation on numerous and various substances, it has now been found that a certain amidinoformic acid derivative or its salt can inhibit strongly and specifically the PAL-activity and exerts a plant growth regulating activity. The present invention is based on this finding.

Accordingly, a main object of this invention is the use of an amidinoformic acid derivative of the formula:

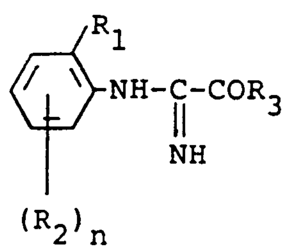

(I)

wherein $R_1$ is a hydrogen atom, a $C_1$-$C_3$ alkyl group, a $C_1$-$C_3$ alkoxy group, a $C_1$-$C_3$ alkylthio group or a halogen atom, $R_2$s are, the same or different, each a $C_1$-$C_3$ alkyl group or a halogen atom, $R_3$ is a hydroxy group, a $C_1$-$C_5$ alkoxy group, a benzyloxy group or a di($C_1$-$C_3$)alkylamino group and n is an integer of 0, 1 or 2, or its salt, as a plant growth regulating agent.

A further object of this invention is to provide a method for regulating the growth of plants, which comprises applying a regulatorily effective amount of an amidinoformic acid derivative of the formula:

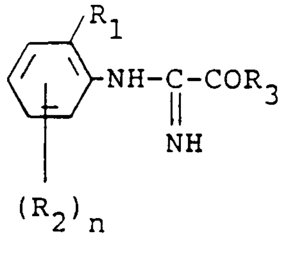

(I)

wherein $R_1$ is a hydrogen atom, a $C_1$-$C_3$ alkyl group, a $C_1$-$C_3$ alkoxy group, a $C_1$-$C_3$ alkylthio group or a halogen atom, $R_2$s are, the same or different, each a $C_1$-$C_3$ alkyl group or a halogen atom, $R_3$ is a hydroxy group, a $C_1$-$C_5$ alkoxy group, a benzyloxy group or a di($C_1$-$C_3$)alkylamino group and n is an integer of 0, 1 or 2, or its salt, to the plants.

In this specification, the term "halogen" includes fluorine, chlorine, bromine and iodine.

Among the amidinoformic acid derivatives encompassed by the formula (I), preferred are those wherein $R_1$ is hydrogen, methyl, ethyl, methoxy or halogen, $R_2$ is methyl, ethyl or halogen, $R_3$ is hydroxy , methoxy , ethoxy , n-propoxy , iso-propoxy or dimethylamino and n is 0 or 1. More preferred are those wherein $R_1$ is hydrogen, methyl, methoxy or halogen, $R_2$ is methyl or halogen, $R_3$ is hydroxy and n is 0 or 1. The most preferred are those wherein $R_1$ is methyl, chlorine or fluorine, $R_2$ is methyl, chlorine or fluorine, $R_3$ is hydroxyl and n is 0 or 1. Specific examples of the preferred compounds are N-(3-fluoro-2-methylphenyl)-amidinoformic acid, N-(3-chloro-2-methylphenyl)amidinoformic acid, N-(5-chloro-2-methylphenyl)-amidinoformic acid, N-(4-fluoro-2-methylphenyl)amidinoformic acid and N-(2-chloro-4-methylphenyl)-amidinoformic acid, or their salts.

As stated above, the amidinoformic acid derivatives (I) or its salt can inhibit the PAL-activity in plants and control the production of secondary metabolites originated from trans-cinnamic acid. For instance, they suppress the production of lignin in trees (e.g. broad-leaved trees, needle-leaf trees), cereals (e.g. wheat, barley, oats) and annual crops (e.g. rice plant, sorghum, corn), so that the utilization of these plants as pulp materials is made possible. Further, they lower the production of lignin in pasture grasses, whereby soft, digestible and nutritious pasture grasses suitable for raising animals are provided. Furthermore, they control the production of chlorogenic acid so that plant seeds of sunflower, soybean, cotton and peanut, tuberous roots of potato and sweet potato, can be prevented from blacking and taste deterioration.

Morevoer, the amidinoformic acid derivatives (I) or its salt can suppress specifically the production of flavonoids in flowers, and therefore flowers having a variety of colors are made available. Representative examples of flowers to which they are applicable are as follows: Japanese morningglory, (common) hydrangea, (common) China aster, anemone, carnation, balloon flower, chrysanthemum, (common) Gypsophilla, crocus, (common) coleus, garbera, narcissus, sweet pea, siebold primrose, (common) stock, tulip, cacti, lilly of the valley, Brassica oleracea L. var. acephara DC, wideleaf sea lavender, garden pansy, corn poppy, Gentiana triflora, (common) petunia, tree paeony, orchids, (common) portulace, Rhododendrons, primroses, lillies, azaleas, (common) macellia, Enkianthus perulatus Schneid and roses.

Besides, the amidinoformic acid derivative (I) can promote the production of such secondary metabolites as biosynthesized from phenylalanine without passing through trans-cinnamic acid as the result of their inhibition of the PAL-activity. Examples of these metabolites are tropane alkaloids (e.g. hyoscyamine, scopolamine), phenanthridine alkaloids (e.g. lycorine, hemanthamine, galanthamine) and colchicines.

Among the amidinoformic acid derivatives (I), some are known (cf. JP-A-108933/78 and 80239/74) and some others are novel. Irrespective of whether they are known or novel, it has never been reported that they have a plant growth regulating activity. This invention thus provides for the first time the use of the amidinoformic acid derivatives (I) as plant growth regulators.

Typical examples of the amidinoformic acid derivatives (I) which are novel are those wherein (a) $R_1$ is ethyl or fluorine, $R_3$ is hydroxy and n is 0, (b) $R_1$ is methyl, $R_2$ is fluorine at the 3-position, fluorine or bromine at the 4-position, fluorine or chlorine at the 5-position or chlorine at the 6-position, $R_3$ is hydroxy and n is 1, (c) $R_1$ is fluorine, $R_2$ is fluorine at the 4-position, $R_3$ is hydroxy and n is 1, (d) $R_1$ is chlorine, $R_2$ is methyl at the 4-position, $R_3$ is hydroxy and n is 1, (e) $R_1$ is methoxy, $R_2$ is chlorine at the 3-position, $R_3$ is hydroxy and n is 1, (f) $R_1$ is hydrogen, $R_2$s are methyl at the 3-position and fluorine at the 4-position, $R_3$ is hydroxy and n is 2 and (g) $R_1$ is methyl, $R_2$ is chlorine at the 3-position, $R_3$ is dimethylamino and n is 1. The provision of these novel compounds may be understood to be an object of the invention, too.

The amidinoformic acid derivatives (I) can be produced by various procedures, of which typical examples are shown below.

Procedure (A):-

The amidinoformic acid derivative of the formula:

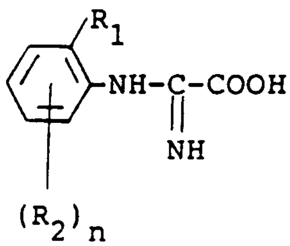

(I-1)

wherein $R_1$, $R_2$ and n are each as defined above is produced by hydrolyzing a compound of the formula:

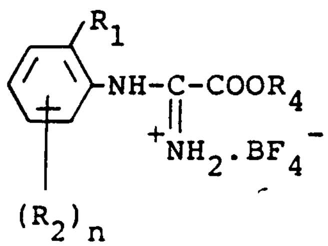

(II)

wherein $R_4$ is a $C_1$-$C_5$ alkyl group or a benzyl group and $R_1$, $R_2$ and n are each as defined above with a base such as an aqueous alkaline solution or a strongly basic ion exchange resin.

When the hydrolysis is performed using an aqueous alkaline solution, the alkali may be sodium hydroxide, potassium hydroxide, potassium carbonate or the like. The amount of the alkali may be 2 to 3 moles to 1 mole of the compound (II). The reaction is normally carried out in a solvent (e.g. water, methanol, ethanol, or a mixture thereof) at a temperature of 0 to 60 °C within a period of 0.5 to 2 hours.

As the strongly basic ion exchange resin, there may be employed Dowex®-1 (1 x 1, $HCO_3^-$) (Dow Chemical Corp.), etc.

Recovery of the product from the reaction mixture may be achieved by a per se conventional procedure such as recrystallization or chromatography on silica gel.

Procedure (B):-

The amidinoformic acid derivative of the formula:

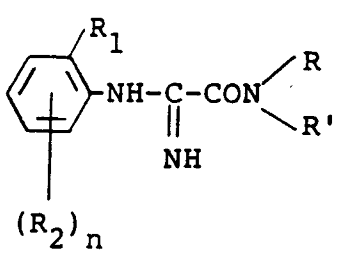

(I-2)

wherein R and R′ are each a $C_1$-$C_3$ alkyl group and $R_1$, $R_2$ and n are each as defined above is produced by reacting the compound (II) with di($C_1$-$C_3$)alkylamine.

The reaction is usually carried out using 2 to 3 moles of di($C_1$-$C_3$)alkylamine to 1 mole of the compound (II). Other reaction conditions may be substantially the same as in Procedure (A).

Procedure (C):-

The amidinoformic acid derivative (I-1) can also be produced by reacting a compound of the formula:

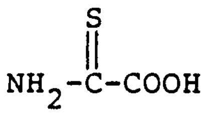

(III)

with a compound of the formula:

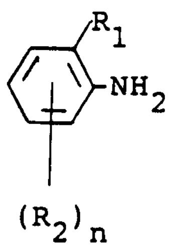

(IV)

wherein $R_1$, $R_2$ and n are each as defined above.

For the reaction, the compound (IV) may be employed in a free form or a salt form with a mineral acid. In the latter case, the mineral acid salt is treated with a base such as an alkali hydroxide prior to the reaction so as to liberate the aniline. The compound (III) and the compound (IV) may be treated in a molar proportion of 1 : 1.0-1.2. The reaction is normally carried out in a solvent such as an alcohol (e.g. methanol, ethanol), a ketone (e.g. acetone) or an ether (e.g. tetrahydrofuran, dioxane), or a mixture thereof, a temperature of 0 to 60 °C, preferably at room temperature, for a period of 0.5 to 12 hours. When desired, a metal oxide or salt (e.g. mercury oxide, silver oxide, lead oxide, lead carbonate, silver carbonate) may be added to the reaction system in an amount of 1 to 10 moles to 1 mole of the compound (III).

Procedure (D):-

The amidinoformic acid derivative of the formula:

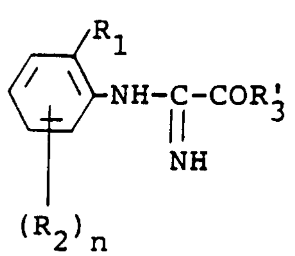

(I-3)

wherein $R_3'$ is a $C_1$-$C_5$ alkoxy group or a benzyloxy group and $R_1$, $R_2$ and n are each as defined above is produced by neutralizing the compound (II) with a base, the amount of which may be usually 1 mole to 1 mole of the compound (II).

The reaction is normally carried out in a solvent such as an alcohol (e.g. methanol, ethanol), a ketone (e.g. acetone), an ether (e.g. tetrahydrofuran, dioxane), a water or a mixture thereof. As the base, there may be used sodium hydroxide, potassium hydroxide, sodium carbonate, potassium carbonate, sodium bicarbonate or an alkali metal alkoxide (e.g. sodium methoxide, sodium ethoxide). After completion of the reaction, the reaction mixture is subjected to post-treatment in a usual manner.

The compound having the formula (I-1) can easily be converted to its salt by reacting it with a base such as sodium hydroxide, potassium hydroxide, sodium methoxide or sodium ethoxide. Typical procedure for conversion of the compound into its salt is as follows.

The compound of the formula (I-1) is dissolved or suspended in a solvent and then one equivalent of the base in the form of a solid or a solution is added thereto under ice-cooling or at room temperature. After being left to stand for 10 minutes to one hour, the resultant solution is subjected to post-treatment such as concentration under reduced pressure and, if necessary, recrystallization. Examples of the solvent are a lower alcohol (e.g. methanol, ethanol), an aromatic hydrocarbon (e.g. toluene, benzene), an ether (e.g. ethyl ether, tetreahydrofuran, dioxane), a halogenated hydrocarbon (e.g. chloroform, dichloromethane, dichloroethane), a hydrocarbon (e.g. hexane), a ketone (e.g. acetone, methyl ethyl ketone), an ester (e.g. ethyl acetate), water, or a mixture thereof.

The compounds having the formulas (I-2) and (I-3) can also easily be converted to their salts by reacting them with an acid such as hydrogen chloride, hydrogen bromide, nitric acid, sulfuric acid, acetic acid or p-toluene sulfonic acid.Typical procedure for conversion of these compounds into their salts is as follows.

The compound of the formula (I-2) or (I-3) is dissolved or suspended in a solvent and then one equivalent of the acid in th form of gaseous, liquid or aqueous solution is added thereto under ice-cooling or at room temperature. After being left to stand for 10 minutes to one hour, the resultant solution is subjected to post-treatment such as concentration under reduced pressure and, if necessary, recrystallization. Examples of the solvent are a lower alcohol (e.g. methanol, ethanol), an aromatic hydrocarbon (e.g. toluene, benzene), an ether (e.g. ethyl ether, tetreahydrofuran, dioxane), a halogenated hydrocarbon (e.g. chloroform, dichloromethane, dichloroethane), a hydrocarbon (e.g. hexane), a ketone (e.g. acetone, methyl ethyl ketone), an ester (e.g. ethyl acetate), water, or a mixture thereof.

Some typical embodiments of production of the amidinoformic acid derivatives (I) are illustratively shown in the following Synthesis Examples.

Synthesis Example 1

To a solution of ethyl thioxamidate (1.0 g) in dichloromethane (10 ml), trimethyloxonium tetrafluoroborate (1.17 g) was added, and the resultant mixture was stirred at room temperature for 30 minutes. 3-Fluoro-2-methylaniline (0.94 g) was added thereto, followed by stirring for 10 minutes. The reaction mixture was concentrated. The residue was dissolved in methanol (10 ml), an aqueous solution (5 ml) of potassium hydroxide (1.05 g) was added thereto, and the resultant mixture was stirred for 30 minutes. The reaction mixture was neutralized with acetic acid (0.5 g) and concentrated under reduced pressure. The solid residue was dissolved in a mixture of dichloromethane and methanol (4 : 1 by volume) (100 ml), followed by filtration. The filtrate was concentrated under reduced pressure, and the oily residue was crystallized from acetone to give N-(3-fluoro-2-methylphenyl)amidinoformic acid (Compound No. 1) (1.15 g).

Synthesis Example 2

To a solution of ethyl thioxamidate (1.0 g) in dichloromethane (10 ml), trimethyloxonium tetrafluoroborate (1.17 g) was added, and the resultant mixture was stirred at room temperature for 30 minutes. 3-Chloro-2-methylaniline (1.1 g) was added thereto, followed by stirring for 10 minutes. The reaction mixture was concentrated. The residue was dissolved in methanol (10 ml), a 40 % aqueous dimethylamine solution (2.1 g) was added thereto, and the resultant mixture was stirred at room temperature for 10 hours. The reaction mixture was concentrated, and the residue was chromatographed on silica gel column using a mixture of dichloromethane and methanol (4 : 1 by volume) as an eluting solvent to give N-(3-chloro-2-methylphenyl)amidinoformic acid N,N-dimethylamide (Compound No. 2) (0.86 g).

Synthesis Example 3

In the same manner as in Example 1, ethyl thioxamidate (1.0 g) was reacted with 3-chloro-2-methylaniline (1.1 g) to give N-(3-chloro-2-methylphenyl)amidinoformic acid (Compound No. 3) (1.25 g).

Synthesis Example 4

In the same manner as in Example 1, ethyl thioxamidate (1.0 g) was reacted with 5-chloro-2-methylaniline (1.1 g) to give N-(5-chloro-2-methylphenyl)amidinoformic acid (Compound No. 4) (1.2 g).

Synthesis Example 5

In the same manner as in Example 1, ethyl thioxamidate (1.0 g) was reacted with 4-fluoro-2-methylaniline (0.94 g) to give N-(4-fluoro-2-methylphenyl)amidinoformic acid (Compound No. 5) (1.05 g).

Synthesis Example 6

To a solution of ethyl thioxamidate (1.0 g) in dichloromethane (10 ml), trimethyloxonium tetrafluoroborate (1.17 g) was added, and the resultant mixture was stirred at room temperature for 30 minutes. 3-Fluoro-2-methylaniline (0.94 g) was added thereto, followed by stirring for 10 minutes. The reaction mixture was concentrated. The mixture was dissolved in methanol (10 ml), 1N sodium bicarbonate (8 ml) was added thereto, and the resultant mixture was stirred at room temperature for 1 hour. The reaction mixture was concentrated, and the residue was chromatographed on silica gel column using a mixture of dichloromethane and methanol (10 : 1 by volume) as an eluting solvent to give ethyl N-(3-fluoro-2-methylphenyl)amidinoformate (Compound No. 26) (0.8 g).

Synthesis Example 7

To a solution of N-(3-chloro-2-methylphenyl)amidinoformic acid N,N-dimethylamide (0.5 g) in ethanol (10 ml) was added 1N hydrogen chloride (2.1 ml). After being left to stand at room temperature for 30 minutes, the solution was concentrated under reduced pressure to obtain hydrochloride of N-(3-chloro-2-methylphenyl)amidinoformic acid N,N-dimethylamide (0.53 g) (Compound No. 29).

Synthesis Example 8

To a suspension of N-(3-chloro-2-methylphenyl)amidonoformic acid (0.5 g) in ethanol (10 ml) was added 1N sodium hydroxide (2.4 ml). After being left to stand at room temperature for 30 minutes, the resultant clear solution was concentrated under reduced pressure to give sodium salt of N-(3-chloro-2-methylphenyl)amidinoformic acid (0.55 g) (Compound No. 30).

In the same manner as above, potassium salt of N-(3-chloro-2-methylphenyl)amidinoformic acid (Compound No. 31) was obtained.

Examples of the amidinoformic acid derivatives (I) produced in the same manner as above are shown in Table 1.

Table 1

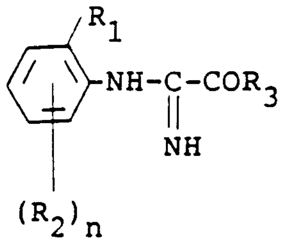

(I)

| Compound No. | 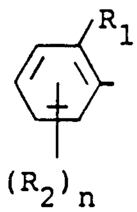 | $R_3$ | Melting point (°C) *1) |
|---|---|---|---|
| 1 | 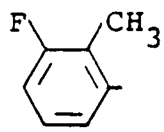 | OH | 155.4 |
| 2 | 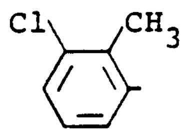 | $N(CH_3)_2$ | 151.6 |
| 3 | 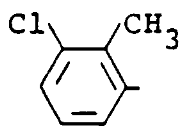 | OH | 154.7 |
| 4 | 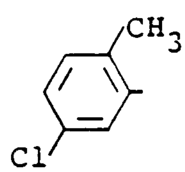 | OH | 152.1 |
| 5 | 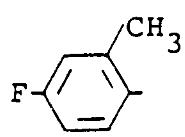 | OH | 188.6 |
| 6 | 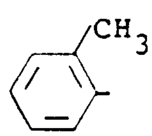 | OH | 143.3 |

(Continued)

| | | | |
|---|---|---|---|
| 7 | 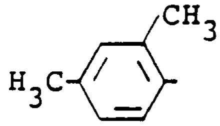 | OH | 159.5 |
| 8 | 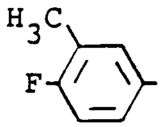 | OH | 197.5 |
| 9 | 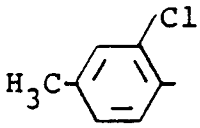 | OH | 147.0 |
| 10 | 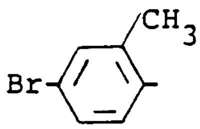 | OH | 167.5 |
| 11 | 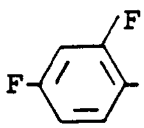 | OH | 200 < |
| 12 | 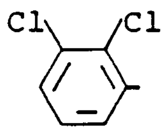 | OH | 137.1 |
| 13 | 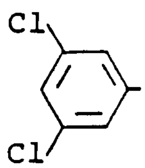 | OH | 131.2 |
| 14 | 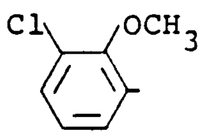 | OH | 131.5 |
| 15 | 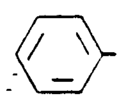 | OH | 150.5 |

(Continued)

| | | | |
|---|---|---|---|
| 16 | 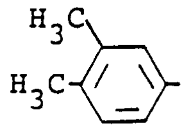 | OH | 145.4 |
| 17 | 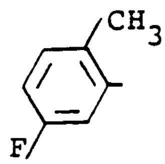 | OH | 149.2 |
| 18 | 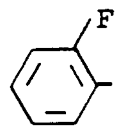 | OH | 122.8 |
| 19 | 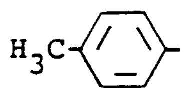 | OH | 188.7 |
| 20 | 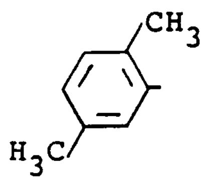 | OH | 151.6 |
| 21 | 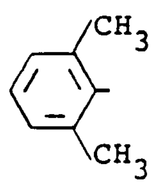 | OH | 165.5 |
| 22 | 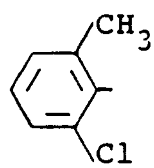 | OH | 107.0 |
| 23 | 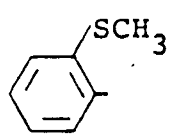 | OH | 134.7 |
| 24 | 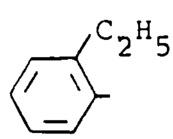 | OH | 91.0 |

(Continued)

| | | | |
|---|---|---|---|
| 25 | 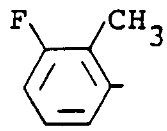 | $OCH_3$ | 135.3 |
| 26 | 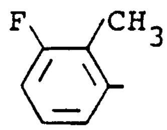 | $OC_2H_5$ | 131.3 |
| 27 | 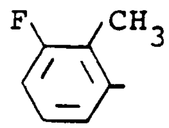 | O-iso-$C_3H_7$ | 127.4 |
| 28*2) | 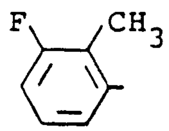 | $OCH_3$ | 145.0 |
| 29*3) | 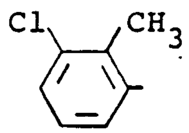 | $N(CH_3)_2$ | 238.0 |
| 30*4) | 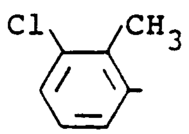 | OH | 300 < |
| 31*5) | 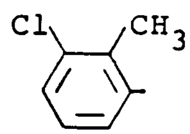 | OH | 253.5 |

Note:
- *1) Decomposition took place with vesication except Compound No. 2.
- *2) Hydrochloride of Compound No. 25.
- *3) Hydrochloride of Compound No. 2.
- *4) Sodium salt of Compound No. 3.
- *5) Potassium salt of Compound No. 3.

The starting compound in Procedures (A) or (B), i.e. Compound (II), is obtainable according to the following reaction scheme:

Step 1

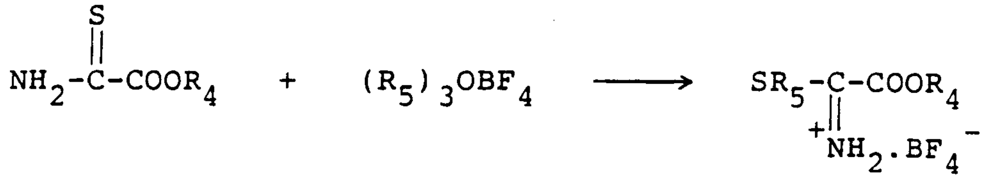

(V) (VI) (VII)

Step 2

(VII)

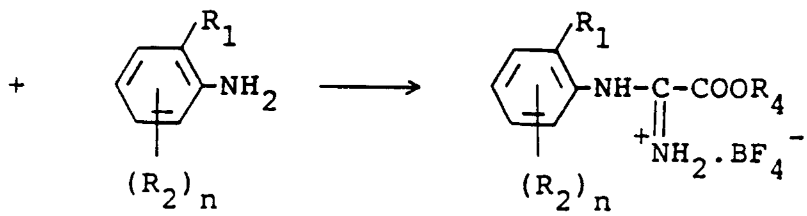

(IV) (II)

wherein $R_5$ is a lower alkyl group (e.g. methyl, ethyl) and $R_1$, $R_2$, $R_4$ and n are each as defined above.

In Step 1, the compound (V) and the compound (VI) may be reacted in a molar ratio of 1:1-1.2, usually in a solvent (e.g. chloroform, dichloromethane, tetrahydrofuran, dioxane, ether, ethyl acetate, benzene, toluene, a mixture thereof) at a temperature of -10 to 60 ° C, preferably at room temperature, for a period of 5 minutes to 2 hours. The product (i.e. Compound (VII)) may be separated from the reaction mixture by a per se conventional procedure or subjected to the reaction in Step 2 without the separation.

In Step 2, the compound (VII) and the compound (IV) may be reacted in a molar ratio of 1:1-1.2, normally in a solvent such as an alcohol or a halogenated hydrocarbon (e.g. dichloromethane, chloroform) at a temperature of -10 to 60 ° C, preferably at room temperature, for a period of 5 minutes to 2 hours. The product (i.e. Compound (II)) may be recovered from the reaction mixture by a per se conventional separation procedure.

For the practical usage of the amidinoformic acid derivative (I) as a plant growth regulator, it is usually formulated with any conventional solid or liquid carrier(s) or diluent(s) as well as any surface active or auxiliary agent(s) into a conventional preparation form such as an emulsifiable concentrate, a solution, a wettable powder, a suspension and a granule. The content of the amidinoformic acid derivative (I) as the active ingredient in such preparation form is normally within a range of about 0.1 to 80 % by weight, preferably of about 1 to 50 % by weight.

Examples of the solid carrier or diluent are fine powders or granules of kaolin clay, attapulgite clay, bentonite, terra alba, pyrophyllite, talc, diatomaceous earth, calcite, walnut powders, urea, ammonium sulfate and synthetic hydrous silicate, etc. As the liquid carrier or diluent, there may be exemplified aromatic hydrocarbons (e.g. xylene, methylnaphthalene), alcohols (e.g. isopropanol, ethylene glycol, cellosolve), ketones (e.g. acetone, cyclohexanone, isophorone), plant oils (e.g. soybean oil, cotton seed oil), dimethylsulfoxide, N,N-dimethylformamide, acetonitrile, water, etc.

The surface active agent usable for emulsification, dispersing or spreading may be of any type, for instance, either anionic or non-ionic. Examples of the surface active agent include alkylsulfates, alkylsulfonates, alkylarylsulfonates, dialkylsulfosuccinates, phosphates of polyoxyethylenealkylaryl ethers, polyoxyethylene alkyl ethers, polyoxyethylene alkylaryl ethers, polyoxyethylene polyoxypropylene block copolymer, sorbitan fatty acid esters, polyoxyethylene sorbitan fatty acid esters, etc. Examples of the auxiliary agents include ligninsulfonates, sodium alginate, polyvinyl alcohol, gum arabic, CMC (carboxymethyl cellulose) and PAP (isopropyl acid phosphate).

Practical embodiments of the plant growth regulating composition according to the present invention are illustratively shown in the following Formulation Examples wherein parts are by weight. The compound number of the active ingredient corresponds to the one as shown in Table 1.

Formulation Example 1

One part of Compound No. 1, 3, 5, 6, 28, 29, 30 or 31, 10 parts of ethylene glycol, 5 parts of polyoxyethylene nonylphenyl ether and 84 parts of water are well mixed to obtain a solution.

Formulation Example 2

Thirty parts of any one of Compounds Nos. 1 to 31, 45 parts of diatomaceous earth, 20 parts of synthetic hydrous silica, 3 parts of sodium laurylsulfate and 2 parts of sodium ligninsulfonate are well mixed while being powdered to obtain a wettable powder.

Formulation Example 3

Ten parts of any one of Compound Nos. 1 to 31, 35 parts of bentonite, 51 parts of fubasami clay, 2 parts of sodium ligninsulfonate, 2 parts of "Sorpol 5060"® (Toho Chemical; anionic surfactant) and 20 parts of water are well mixed, and the mixture is granulated and dried to obtain granules.

The composition containing the amidinoformic acid derivative (I) thus formulated may be applied by any appropriate procedure for growth control of plants. Typical application procedures are soil treatment, foliar spray, addition to solution in water culture, immersion of seeds or bulbs, injection to plant stems, surface coating on plants and addition to medium for tissue culture.

When, for instance, the control of the lignin production is aimed at, the composition may be periodically applied to trees such as broad-leaved trees and needle-leaved trees at the young stage by foliar spray, soil treatment, injection to the stems or surface coating on stems. For cereals (e.g. wheat, barley, oats) and annual plants (e.g. rice plant, sorghum, corn), the application at the stage of seedlings by foliar spray or soil treatment is desirable. For pasture grasses, the application at the stage of seedlings by foliar spray or soil treatment is likewise desirable, but the application may be also made by immersion of seeds.

When the suppression of the chlorogenic acid production is aimed at, the target plants (e.g. sunflower, cotton, peanut) may be treated with the composition by foliar spray or soil treatment at the stage between termination of blooming and beginning of seed-setting. In case of the target plants being tuberous or rooted (e.g. potato, sweet potato), treatment may be made by foliar spray and soil drench before growth of tubers or roots.

In order to decrease the flavonoid production, the application may be performed by foliar spray or soil treatment at the stage from differentiation of flower bud to blooming. Immersion of bulbs is also applicable. In order to promote the production of tropane alkaloids, phenanthridine alkaloids or colchicines, tissue culture such as hairy roots may be effected in a medium containing the composition.

The amidinoformic acid derivative (I) may be used together with herbicides or other plant growth regulators to improve its activity as a plant growth regulator, and in some cases, a synergistic effect can be expected.

The dosage of the amidinoformic acid derivative (I) may vary depending on the treating condition, kind and species of plants, growing stage and prevailing season. Generally, the concentration of the active ingredient in foliar spray is from about 1 to 8,000 ppm, preferably from about 50 to 4,000 ppm. In case of soil treatment, the active ingredient may be used in an amount of about 10 to 4,000 grams, preferably of about 100 to 2,000 grams, per hectare. In water culturing, the concentration may be from about 0.01 to 100 ppm, preferaby from about 0.1 to 10 ppm. Seed immersion may be performed in a concentration of about 0.1 to 4,000 ppm, preferably of about 1 to 1000 ppm. The dosage in injection to plants is normally from about 1 mg to 40 g, preferably from about 10 mg to 10 g per tree, whereas surface coating onto plants may be made in a concentration of about 10 to 100,000 ppm, preferably of about 1,000 to 10,000 ppm. When used in tissue cultivation, the concentration of the active ingredient to be added to a culture medium is from about 0.01 to 100 ppm, preferably from about 0.1 to 10 ppm. Likewise, immersion of bulbs may be performed in a concentration of about 0.1 to 4,000 ppm, preferably of about 10 to 1,000 ppm.

The biological test data of the amidinoformic acid derivative (I) as a plant growth regulator will be illustratively shown in the following Examples.

Test Example 1

Inhibitory effect on PAL-activity:-

A potato (var.: May queen) was sliced in 1 to 2 mm thick and cultivated at 25 °C for 30 hours under 8000 luxes, followed by homogenization in 0.1 M borate buffer. Fractions thus obtained were filtered with a piece of cheese-cloth and centrifuged to precipitate the residue. Ammonium sulfate was added to the supernatant liquid so as to make a 30 % saturated concentration, followed by centrifugation to precipitate waste materials. Addition of ammonium sulfate was repeated to adjust the supernatant liquid at a 70 % saturated concentration, followed by centrifugation. The precipitate was dissolved in 0.1 M borate buffer to make a crude PAL-enzyme solution. To a designated amount of the PAL-enzyme solution, phenylanaline as a substrate and a designated amount of the compound (I) were added, and the resultant mixture was allowed to react at 37 °C for 1 hour. The amount of trans-cinnamic acid produced was calculated by measuring an absorption at 290 nm on a spectrophotometer. The concentration of the compound (I) which inhibits 50 % the PAL-activity is shown in Table 2.

Table 2

| Compound No. | Concentration of 50 % inhibition of PAL-activity (M) |
|---|---|
| 1 | $9 \times 10^{-8}$ |
| 3 | $7 \times 10^{-8}$ |
| AOPP | $7 \times 10^{-7}$ |

Test Example 2

Inhibitory effect on antocyanin production:-

Water culture (10 ml) containing the compound (I) was charged in a reaction vessel, and on adsorbed cotton laid down therein 15 seeds of red cabbase (var.: Ruby Ball) were sowed and cultivated for 5 days at 25 °C under 8000 luxes. The shoot of the germed cabbage was cut and antocyanin was extracted with 1N hydrochloric acid (5 ml). The amount of antocyanin thus extracted was calculated by meauring an absorption at 535 nm on a spectrophotometer. The concentration of the compound (I) which inhibits 50 % the antocyanin production was calculated in contrast to the non-treated plot. The results are shown in Table 3. Still, neither abnormality in the vegetative part nor suppression of growth of the test plant due to Compound (I) could be observed.

Table 3

| Compound No. | Concentration of 50 % inhibition of antocyanin production (ppm) |
|---|---|
| 1 | 0.04 |
| 3 | 0.02 |
| 4 | 0.1 |
| 5 | 0.1 |
| 6 | 0.3 |
| 7 | 0.9 |
| 8 | 0.2 |
| 9 | 0.1 |
| 10 | 0.3 |
| 11 | 0.4 |
| 12 | 0.2 |
| 13 | 0.4 |
| 14 | 0.2 |
| 15 | 3 |
| 16 | 3 |
| 17 | 1 |
| 18 | 3 |
| 19 | 3 |
| 25 | 0.06 |
| 26 | 0.06 |
| 27 | 0.08 |
| 28 | 0.06 |
| 30 | 0.05 |
| 31 | 0.05 |
| AOPP | 4 |

Test Example 3

Promoting effect on scopolamine production:-

Hairy root of Duboisia leichhardtii are known to produce scopolamine once vegetative parts were infected with Agrobacterium rhizogenes (Plant Science, 59, 191 - 201 (1989)). One hairy root of Duboisia - (var.: DL47-1) (wet content, 100 mg) was inoculated in a liquid HF-3 medium (50 ml) and subjected to rotation-culture with a speed of 85 rotations/min at 25 °C in the dark place. After twenty-one days, 1000-fold dilution of a dimethylsulfoxide solution of Compound (I) was added to the culture medium to make its final concentration constant. Twenty-eight days thereafter, scopolamine in the hairy roots and the medium was respectively extracted and its amount was measured on a high performance liquid chromatography (HPLC). The amount of scopolamine per culture (50 ml) is shown in Table 4.

Table 4

| Compound No. | Final concentration (mg/l) | Amount of scopolamine (mg) | | |
|---|---|---|---|---|
| | | Root | Medium | Total |
| 1 | 0.1 | 2.1 | 2.7 | 4.8 |
| | 1.0 | 2.4 | 3.4 | 5.8 |
| Non-treated | - | 1.9 | 1.7 | 3.6 |

Test Example 4

Inhibitory effect on antocyanin production in morningglory:-

Seeds of morningglory (var.: Scarlet Ohara) was sowed in small pots (diameter, 8 cm) and cultivated in a greenhouse. At the time of development of cotyledons (9 days after sowing), short-day treatment was performed for 1 week to induce flowering. Five days prior to flowering (length of buds being about 1 to 2 cm), a designated amount of the compound (I) was applied to the soil or sprayed to the foliage of the plant. Cultivation was continued, and color of petals was visually observed in contrast to the non-treated plot and evaluated into five indices ranging from (-: no influence) to (+ + + +: completely whitened). The results are shown in Table 5.

Table 5

| Treating method | Compound No. | Final concen-centration | Color index |
|---|---|---|---|
| Soil treat-ment | 1 | 2 mg/pot | +++ |
| | 3 | 2 " | +++ |
| | | 0.5 " | ++ |
| | 4 | 2 " | +++ |
| | 5 | 2 " | +++ |
| | 6 | 4 " | +++ |
| | AOPP | 10 " | - |
| Foliar treat-ment | 1 | 2000 ppm | +++ |
| | 3 | 1000 " | +++ |
| | 5 | 1000 " | +++ |
| | AOPP | 2000 " | - |

As understood from the above test results, no adverse effect was observed in the plants treated with Compound (I). In contrast, AOPP induced severe phytotoxicity (withering of leaves) both by soild and foliar spray treatments.

Test Example 5

Inhibitory effect on lignin production in wheat and softening of wheat culm:-

Wheat seeds (var.: Nohrin No. 61) were sowed in small pots (6 seeds/pot) and cultivated in a greenhouse for about one month. When differentiation of culm was recognized, a designated amount of the compound in the form of a wettable powder according to Formulation Example 2 was applied to the soil, and cultivation was continued until blooming. Hardness of wheat culms in contrast to those of untreated was manually determined and evaluated into four indices ranging from (-: unchanged) to (+ + +: prominently softened). The results are shown in Table 6.

Table 6

| Compound No. | Concentration (mg/pot) | Softening index |
|---|---|---|
| 1 | 4<br>2 | + + +<br>+ + |
| 3 | 2<br>1 | + + +<br>+ + |
| Non-treated | 0 | - |

Test Example 6

Inhibitory effect on chlorogenic acid production in sunflower:-

Sunflowers (var.: Russian Sunflower) were grown in a greenhouse for two weeks and transplanted in Wagner's pots (1/5000 are) and cultivated for 2 months outdoors. When 5 to 10 days elapsed after blooming, a designated amount of the compound in the form of a wettable powder according to Formulation Example 2 was sprayed over the flowers, from which seeds were harvested. Endosperm of the seeds were crushed and subjected to boiling-extraction with ethanol and filtration. The filtrate was diluted with 0.05 M aqueous $H_3PO_4$ and further filtered. The amount of chlorogenic acid was quantitatively analysed by HPLC (column: Nucleosil C18 (8 mm x 25 cm); eluent: methanol : 0.05 M $H_3PO_4$ (35 : 75)). The results are shown in Table 7.

Table 7

| Compound No. | Concentration (ppm) | Content of chlorogenic acid (μg/mg fr.wt) |
|---|---|---|
| 1<br>3 | 1000<br>1000 | 1.3<br>1.0 |
| Non-treated | - | 3.4 |

Still, it may be apparent to those skilled in the art that the amidinoformic acid derivative (I) can also be representable by the formula:

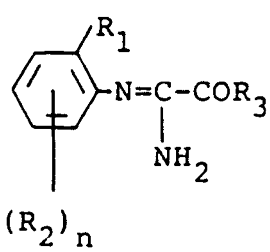

wherein $R_1$, $R_2$, $R_3$ and n are each as defined above.

## Claims

1. Use of an amidinoformic acid derivative of the formula:

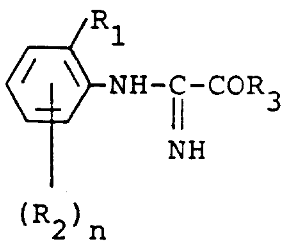

(I)

wherein $R_1$ is a hydrogen atom, a $C_1$-$C_3$ alkyl group, a $C_1$-$C_3$ alkoxy group, a $C_1$-$C_3$ alkylthio group or a halogen atom, $R_2$s are, the same or different, each a $C_1$-$C_3$ alkyl group or a halogen atom, $R_3$ is a hydroxy group, a $C_1$-$C_5$ alkoxy group, a benzyloxy group or a di($C_1$-$C_3$)alkylamino group and n is an integer of 0, 1 or 2, or its salt, as a plant growth regulating agent.

2. A method for regulating the growth of plants which comprises applying a regulatorily effective amount of an amidinoformic acid derivative of the formula:

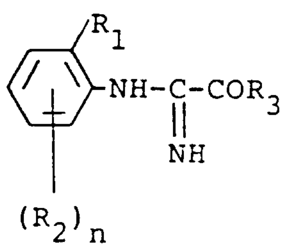

(I)

wherein $R_1$ is a hydrogen atom, a $C_1$-$C_3$ alkyl group, a $C_1$-$C_3$ alkoxy group, a $C_1$-$C_3$ alkylthio group or a halogen atom, $R_2$s are, the same or different, each a $C_1$-$C_3$ alkyl group or a halogen atom, $R_3$ is a hydroxy group, a $C_1$-$C_5$ alkoxy group, a benzyloxy group or a di($C_1$-$C_3$)alkylamino group and n is an integer of 0, 1 or 2, or its salt, to the plants.

3. The method according to claim 2, wherein the activity of phenylalanine ammonia-lyase is inhibited.

4. The method according to claim 3, whereby the production of a secondary metabolite from trans-cinnamic acid via the shikimic acid pathway is lowered.

5. The method according to claim 3, whereby the production of a secondary metabolite from phenylalanine without passing through trans-cinnamic acid is promoted.

6. An amidinoformic acid derivative of the formula:

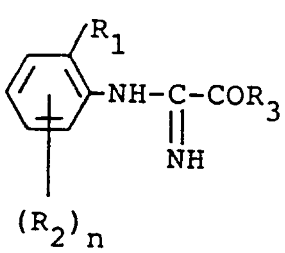

(I)

wherein (a) $R_1$ is ethyl or fluorine, $R_3$ is hydroxy and n is 0, (b) $R_1$ is methyl, $R_2$ is fluorine at the 3-position, fluorine or bromine at the 4-position, fluorine or chlorine at the 5-position or chlorine at the 6-position, $R_3$ is hydroxy and n is 1, (c) $R_1$ is fluorine, $R_2$ is fluorine at the 4-position, $R_3$ is hydroxy and n is 1, (d) $R_1$ is chlorine, $R_2$ is methyl at the 4-position, $R_3$ is hydroxy and n is 1, (e) $R_1$ is methoxy, $R_2$ is chlorine at the 3-position, $R_3$ is hydroxy and n is 1, (f) $R_1$ is hydrogen, $R_2$s are methyl at the 3-position and fluorine at the 4-position, $R_3$ is hydroxy and n is 2 and (g) $R_1$ is methyl, $R_2$ is chlorine at the 3-position, $R_3$ is dimethylamino and n is 1, or its salt.

**7.** The amidinoformic acid derivative according to claim 6, wherein $R_1$ is methyl, $R_2$ is fluorine at the 3-position, $R_3$ is hydroxy and n is 1, or its salt.

**8.** The amidinoformic acid derivative according to claim 6, wherein $R_1$ is methyl, $R_2$ is fluorine at the 4-position, $R_3$ is hydroxy and n is 1, or its salt.

**9.** The amidinoformic acid derivative according to claim 6, wherein $R_1$ is methyl, $R_2$ is chlorine at the 3-position, $R_3$ is hydroxy and n is 1, or its salt.

**10.** The amidinoformic acid derivative according to claim 6, wherein $R_1$ is chlorine, $R_2$ is methyl at the 4-position, $R_3$ is hydroxy and n is 1, or its salt.

**Patentansprüche**

**1.** Verwendung eines Amidinoameisensäure-Derivats der Formel:

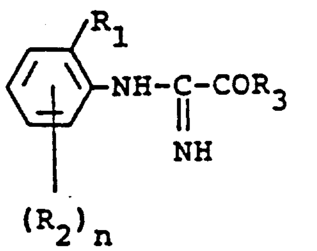

(I)

in der $R_1$ ein Wasserstoffatom, einen $C_1$-$C_3$-Alkylrest, einen $C_1$-$C_3$-Alkoxyrest, einen $C_1$-$C_3$-Alkylthiorest oder ein Halogenatom bedeutet, die Reste $R_2$ gleich oder verschieden sind und jeweils einen $C_1$-$C_3$-Alkylrest oder ein Halogenatom bedeuten, $R_3$ eine Hydroxylgruppe, einen $C_1$-$C_5$-Alkoxyrest, einen Benzyloxyrest oder einen Di($C_1$-$C_3$)alkylaminorest bedeutet und n eine ganze Zahl von 0, 1 oder 2 ist, oder dessen Salzes, als ein Mittel zur Regulation des Pflanzenwachstums.

**2.** Verfahren zur Regulation des Wachstums von Pflanzen, umfassend die Anwendung einer regulatorisch wirksamen Menge eines Amidinoameisensäure-Derivats der Formel:

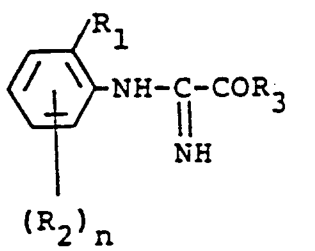

(I)

in der $R_1$ ein Wasserstoffatom, einen $C_1$-$C_3$-Alkylrest, einen $C_1$-$C_3$-Alkoxyrest, einen $C_1$-$C_3$-Alkylthiorest oder ein Halogenatom bedeutet, die Reste $R_2$ gleich oder verschieden sind und jeweils einen $C_1$-$C_3$-Alkylrest oder ein Halogenatom bedeuten, $R_3$ eine Hydroxylgruppe, einen $C_1$-$C_5$-Alkoxyrest, einen Benzyloxyrest oder einen Di($C_1$-$C_3$)Alkylaminorest bedeutet und n eine ganze Zahl von 0, 1 oder 2 ist, oder dessen Salzes, auf die Pflanzen.

3. Verfahren gemäß Anspruch 2, wobei die Wirkung der Phenylalanin-Ammoniak-Lyase gehemmt wird.

4. Verfahren gemäß Anspruch 3, wobei die Bildung eines sekundären Stoffwechselprodukts aus trans-Zimtsäure über den Shikimisäure-Weg vermindert wird.

5. Verfahren gemäß Anspruch 3, wobei die Bildung eines sekundären Stoffwechselprodukts aus Phenylalanin ohne den Weg über trans-Zimtsäure gefördert wird.

6. Amidinoameisensäure-Derivat der Formel:

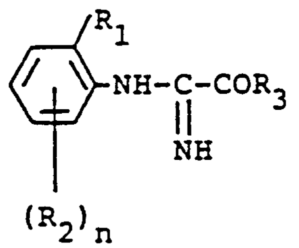

(I)

in der (a) $R_1$ eine Ethylgruppe oder ein Fluoratom bedeutet, $R_3$ eine Hydroxylgruppe bedeutet und n 0 ist, (b) $R_1$ eine Methylgruppe bedeutet, $R_2$ ein Fluoratom in 3-Position, ein Fluor- oder Bromatom in 4-Position, ein Fluor- oder Chloratom in 5-Position oder ein Chloratom in 6-Position bedeutet, $R_3$ eine Hydroxylgruppe bedeutet und n 1 ist, (c) $R_1$ ein Fluoratom bedeutet, $R_2$ ein Fluoratom in 4-Position bedeutet, $R_3$ eine Hydroxylgruppe bedeutet und n 1 ist, (d) $R_1$ ein Chloratom bedeutet, $R_2$ eine Methylgruppe in 4-Position bedeutet, $R_3$ eine Hydroxylgruppe bedeutet und n 1 ist, (e) $R_1$ eine Methoxygruppe bedeutet, $R_2$ ein Chloratom in 3-Position bedeutet, $R_3$ eine Hydroxylgruppe bedeutet und n 1 ist, (f) $R_1$ ein Wasserstoffatom bedeutet, die Reste $R_2$ Methylgruppen in 3-Position und Fluoratome in 4-Position bedeuten, $R_3$ eine Hydroxylgruppe bedeutet und n 2 ist und (g) $R_1$ eine Methylgruppe bedeutet, $R_2$ ein Chloratom in 3-Position bedeutet, $R_3$ eine Dimethylaminogruppe und n 1 ist, oder dessen Salz.

7. Amidinoameisensäure-Derivat gemäß Anspruch 6, worin $R_1$ eine Methylgruppe bedeutet, $R_2$ ein Fluoratom in 3-Position bedeutet, $R_3$ eine Hydroxylgruppe bedeutet und n 1 ist, oder dessen Salz.

8. Amidinoameisensäure-Derivat gemäß Anspruch 6, worin $R_1$ eine Methylgruppe bedeutet, $R_2$ ein Fluoratom in 4-Position bedeutet, $R_3$ eine Hydroxylgruppe bedeutet und n 1 ist, oder dessen Salz.

9. Amidinoameisensäure-Derivat gemäß Anspruch 6, worin $R_1$ eine Methylgruppe bedeutet, $R_2$ ein Chloratom in 3-Position bedeutet, $R_3$ eine Hydroxylgruppe bedeutet und n 1 ist, oder dessen Salz.

10. Amidinoameisensäure-Derivat gemäß Anspruch 6, worin $R_1$ ein Chloratom bedeutet, $R_2$ eine Methylgruppe in 4-Position bedeutet, $R_3$ eine Hydroxylgruppe bedeutet und n 1 ist, oder dessen Salz.

## Revendications

1. Utilisation d'un dérivé d'acide amidinoformique de formule :

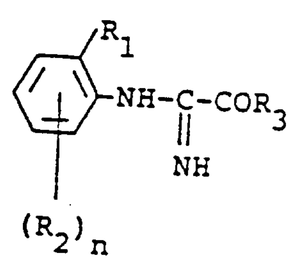

(I)

dans laquelle $R_1$ est un atome d'hydrogène, un groupement alkyle en $C_1$-$C_3$, un groupement alcoxy en $C_1$-$C_3$, un groupement alkylthio en $C_1$-$C_3$ ou un atome d'halogène, les radicaux $R_2$, identiques ou différents, sont chacun un groupement alkyle en $C_1$-$C_3$ ou un atome d'halogène, $R_3$ est un groupement hydroxyle, un groupement alcoxy en $C_1$-$C_5$, un groupement benzyloxy ou un groupement di(alkyle en $C_1$-$C_3$)amino et n est un nombre entier égal à 0, 1 ou 2, ou de son sel, comme agent de régulation de la croissance de végétaux.

**2.** Procédé de régulation de la croissance de végétaux qui comprend l'application aux végétaux d'une quantité régulatrice efficace d'un dérivé d'acide amidino-formique de formule :

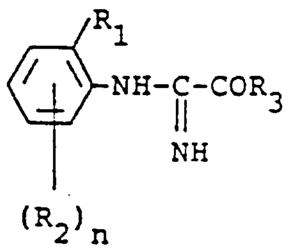

(I)

dans laquelle $R_1$ est un atome d'hydrogène, un groupement alkyle en $C_1$-$C_3$, un groupement alcoxy en $C_1$-$C_3$, un groupement alkylthio en $C_1$-$C_3$ ou un atome d'halogène, les radicaux $R_2$, identiques ou différents, sont chacun un groupement alkyle en $C_1$-$C_3$ ou un atome d'halogène, $R_3$ est un groupement hydroxyle, un groupement alcoxy en $C_1$-$C_5$, un groupement benzyloxy ou un groupement di(alkyle en $C_1$-$C_3$)amino et n est un nombre entier égal à 0, 1 ou 2, ou de son sel.

**3.** Procédé selon la revendication 2, dans lequel on inhibe l'activité de la phénylalanine-désammoniase.

**4.** Procédé selon la revendication 3, dans lequel on réduit la production d'un métabolite secondaire à partir d'acide trans-cinnamique par la voie de l'acide shikimique.

**5.** Procédé selon la revendication 3, dans lequel on facilité la production d'un métabolite secondaire à partir de phénylalanine sans passer par un acide trans-cinnamique.

**6.** Dérivé d'acide amidinoformique de formule :

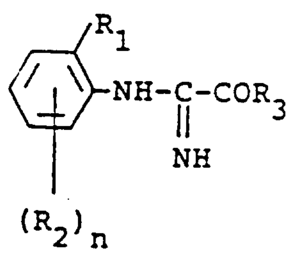

(I)

dans laquelle (a) $R_1$ est un groupement éthyle ou un atome de fluor, $R_3$ est un groupement hydroxyle et n vaut 0, (b) $R_1$ est un groupement méthyle, $R_2$ est un atome de fluor en position 3, un atome de fluor ou de brome en position 4, un atome de fluor ou de chlore en position 5 ou un atome de chlore en position 6, $R_3$ est un groupement hydroxyle et n vaut 1, (c) $R_1$ est un atome de fluor, $R_2$ est un atome de fluor en position 4, $R_3$ est groupement hydroxyle et n vaut 1, (d) $R_1$ est un atome de chlore, $R_2$ est groupement méthyle en position 4, $R_3$ est un groupement hydroxyle et n vaut 1, (e) $R_1$ est un groupement méthoxy, $R_2$ est un atome de chlore en position 3, $R_3$ est un groupement hydroxyle et n vaut 1, (f) $R_1$ est un atome d'hydrogène, les radicaux $R_2$ sont un groupement méthyle en position 3 et un atome de fluor en position 4, $R_3$ est un groupement hydroxyle et n vaut 2 et (g) $R_1$ est un groupement méthyle, $R_2$ est un atome de chlore en position 3, $R_3$ est un groupement diméthylamino et n vaut 1, ou son sel.

7. Dérivé d'acide amidinoformique selon la revendication 6, dans lequel $R_1$ est un groupement méthyle, $R_2$ est un atome de fluor en position 3, $R_3$ est un groupement hydroxyle et n vaut 1, ou son sel.

8. Dérivé d'acide amidinoformique selon la revendication 6, dans lequel $R_1$ est un groupement méthyle, $R_2$ est un atome de fluor en position 4, $R_3$ est un groupement hydroxyle et n vaut 1, ou son sel.

9. Dérivé d'acide amidinoformique selon la revendication 6, dans lequel $R_1$ est un groupement méthyle, $R_2$ est un atome de chlore en position 3, $R_3$ est un groupement hydroxyle et n vaut 1, ou son sel.

10. Dérivé d'acide amidinoformique selon la revendication 6, dans lequel $R_1$ est un atome de chlore, $R_2$ est un groupement méthyle en position 4, $R_3$ est un groupement hydroxyle et n vaut 1, ou son sel.